(19) Europäisches Patentamt
European Patent Office
Office européen des brevets

(11) **EP 2 196 196 A1**

(12) **EUROPEAN PATENT APPLICATION**

(43) Date of publication:
**16.06.2010 Bulletin 2010/24**

(51) Int Cl.:
*A61K 9/50* (2006.01)    *A61K 9/51* (2006.01)
*A61K 9/16* (2006.01)    *A61K 39/39* (2006.01)

(21) Application number: **08171263.0**

(22) Date of filing: **10.12.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | • **Harwood, Lisa**<br>**3072 Ostermundigen (CH)**<br>• **Rossi, Nathanaël**<br>**1110 Morges (CH)** |
| (71) Applicant: **Medipol S.A.**<br>**1015 Lausanne (CH)** | (74) Representative: **Schneiter, Sorin et al**<br>**Abrema Agence Brevets & Marques**<br>**Ganguillet**<br>**Avenue du Théâtre 16**<br>**P.O. Box 5027**<br>**1002 Lausanne (CH)** |
| (72) Inventors:<br>• **McCullough, Ken**<br>**1774 Cousset (CH)**<br>• **Kaeuper, Peter**<br>**1003 Lausanne (CH)** | Remarks:<br>Claims16 to 29are deemed to be abandoned due to non-payment of the claims fees (Rule 45(3) EPC). |

(54) **Compound, medicament, vaccine composition and nanocapsules**

(57)    The present invention relates to a compound comprising a polyelectrolyte and, covalently linked thereto, an immunological adjuvant and/or cell targeting ligand, wherein the covalently linked entity can have both adjuvant and cell targeting characteristics. The compound is used in the preparation of hydrophilic vaccine nanoparticles, which preferably have an antigenic compound or therapeutic agent, or genetic information encoding such compounds or agents entrapped in their matrix, or covalently linked to their surfaces. Vaccine compositions comprising the particles of the invention are advantageous, because a strong and long-lasting immune response is obtained following administration of a single dose. In a preferred embodiment, the polyelectrolyte of the compound is an anionic polymer, and the particle comprises a matrix comprising chitosan.

**Figure 1**

EP 2 196 196 A1

**Description**

**Technical Field**

The present invention relates to the general fields of pharmaceutical sciences, drug delivery, immunotherapy, immunoprophylaxis and vaccinology, in particular to the fields of immunology, vaccines, vaccine delivery, adjuvants and immunotherapy. Furthermore, the present invention relates to the fields of polymer science, colloid science, polyelectrolyte chemistry, and biomedical engineering. The present invention relates to hydrophilic, colloid particles that are useful as carriers of active principles, as medicaments, and in particular as vaccines. The present invention also relates to adjuvants, targeting moieties and/or carriers of immunomodulatory substances for immunotherapeutic and immunoprophylactic applications.

More specifically, the present invention relates to a compound comprising a polyelectrolyte and, covalently connected thereto, a cell-interacting entity, to a colloid particle comprising the compound, to a pharmaceutical composition, to a method of vaccination and to processes for preparing colloid particles.

**Prior Art and the Problem Underlying the Invention**

In the fields of pharmaceutical sciences, vaccinology and immunology, the administration of an active principle is preceded by detailed studies concerning the determination of the optimal amount of the active principle that needs to be administered, the most efficient and/or convenient form of administration, the overall administration interval and so forth, all of which have the final goal of achieving a reliable and desired effect, such as the prevention and/or treatment of an undesired condition, such as a disease. One tenet in this context is that the amount of the active principle and also the overall administration interval in case that the active principle has to be administered is to be as small or short, respectively, as possible. One reason is, of course, cost, since many active principles are obtained in complex and expensive manufacturing processes. Another reason is the avoidance of potential side effects, which may be associated with the administration of an active principle. Accordingly, in the fields of the present invention, it is an objective to increase efficacy and efficiency of drug or vaccine administration.

The development of vaccination compositions and procedures is an example. With respect to vaccines, there is an objective of producing a strong, preferably long-lasting immunisation following one or a limited number of administrations of a vaccine. When one considers that vaccines are generally administered by specialized personal under conditions aimed at preventing introduction of extraneous agents, which would otherwise result in disease or compromisation of the host physiology, it is clearly desirous to reduce the number of individual vaccination interventions necessary to obtain a given protective effect. Repeated vaccinations require repeated medical consultations and involve increased costs. Accordingly, it is an objective of the present invention to achieve a strong immune response following a first vaccination, or a primary followed by booster vaccination (primo-boost) regime.

Saupe et al. "Immunostimulatory colloidal delivery systems for cancer vaccines", informa healthcare, 2006, pp. 345-54, provide a review article on micro- and nanoparticles, liposomes archeosomes and virus-like particles as delivery systems for vaccines against cancer cells. Although the authors discuss the possibilities of controlled release and protection of agents from degradation, this reference is purely concerned with vaccines as therapy for the treatment of cancer. Furthermore, it is mentioned that only few clinical trials that use immunostimulatory colloidal delivery systems have been undertaken, and that existing data shows little vaccine efficacy.

Alving "Liposomes as carriers of antigens and adjuvants", Journal of Immunological Methods, 140 (1991) reports that liposomes are successful for producing immunity *in vivo*. It is pointed out that liposomes interact with macrophages, which makes liposomes suitable as carriers of antigens. On the other hand, liposomes are characterised by high production costs, by occasional leakage of entrapped material and instability issues shortening the shelf life of liposomal formulations. Therefore, it is an objective of the present invention to provide for a vaccine that can be prepared under mild conditions and which thereby benefits the encapsulation of antigens, such as proteins.

Seferian et al. "Immune stimulating activity of two new chitosan containing adjuvant formulations", Vaccine 19 (2001), 661-8 point out the problem of antigen proteins that are only weakly immunogenic. As an adjuvant, a chitosan-based emulsion and a zinc-chitosan particle designed to bind a histidine-tagged recombinant protein is disclosed. While the authors build on the known immune stimulating activity of chitosan, the results indicate that it is irrelevant whether the chitosan adjuvant is administered as a particle or in the form of an emulsion.

Borges et al. "Evaluation of the immune response following a short oral vaccination schedule with hepatitis B antigen encapsulated into alginate-coated chitosan nanoparticles", European Journal of Pharmaceutical Sciences 32 (2007) 278-290, disclose a hepatitis B vaccine, encapsulated in alginate-coated chitosan nanoparticles. In one setting, the antigen was administered in combination with an adjuvant oligodeoxynucleotide (CpG ODN). Following two immunizations, the groups receiving the nanoparticle-associated vaccine antigen showed enhanced immune responsiveness. However, this enhancement was most obvious in terms of a non-specific response to polyclonal activation of T lymphocytes by concanavalin A, which is irrelevant to vaccine design and immune defence induction. Of particular concern is the observation of the authors that while nanoparticle-associated vaccine antigen also enhanced an antigen-specific secondary recall immune response, this was less efficient compared to the recall response obtained with cells from animals vaccinated with antigen in the absence of nanoparticles. The immunological value of the whole analyses is seriously damaged by their observations that the non-vaccinated control animals provided cells which gave a stronger antigen-specific recall response than the animals receiving the nanoparticle-associated vaccine antigen. Moreover, in this setting, the adjuvant did not have any particular beneficial effect.

In view of the above, it is an objective of the present invention to provide a new technology for administration of active principles, applicable to many types of active principles, such as antigens, allergens, adjuvants, immunoregulatory and/or immunomodulatory substances in general, drugs, nucleic acids (such as DNA and RNA). It is an objective to increase the efficacy and/or efficiency of administration of an active principle.

It is an objective of the present invention is to provide a new delivery technology for active principles. Preferably, the new delivery technology has increased efficacy and/or efficiency, is more specific and/or allows for reduction of the administration dose, the administration frequency and/or the overall administration interval (for example, the period of treatment).

The present invention addresses the problem of providing a delivery system that can be prepared under mild, predominantly aqueous conditions thereby preserving the functionality and/or integrity of active principles. In other words, the present invention relates to an efficient administration form which can be used for the administration of active principles that are sensible to organic solvents, or to harsh external conditions such as elevated temperatures, pH values far from neutral, radiation and the like.

It is an objective of the invention to provide a delivery system capable of targeting and/or addressing a target structure, such as, for example, a target tissue, organ and/or target cells of an individual. It is an objective to target an active principle to said target structure.

More specifically, it is an objective of the present invention, to target cells of the immune system, preferably cells of the innate immune system, for example antigen presenting cells and inflammatory mediators represented by dendritic cells and macrophages and neutrophils, respectively. It is an objective to target cells via receptors found particularly on cells, such as the immature dendritic cells referred to as "professional antigen presenting cells" or activated inflammatory immune cells.

It is a further objective of the present invention to exploit the advantage of particulate administration forms, while at the same time optimising the beneficial impact of targeting structures and/or adjuvants.

A further objective of the present invention is to provide a vaccine technology, which is preferably applicable to different antigens and/or adjuvants, and which therefore can be adapted to a variety of specific conditions and/or diseases. Accordingly, it is an objective of the present invention is to prevent or treat diseases by vaccination.

With respect to vaccination, it is an objective of the present invention is to obtain a strong and preferably long-lasting immune response following a primary vaccination or prime-boost regime with a single booster, thus reducing the need for excessive numbers of vaccinations.

The present invention further addresses the problem of preventing or treating a condition and/or disease in general, for example disease associated with a malfunctioning of the immune system, and inflammatory diseases.

## Summary of Invention

The inventors of the present invention provided a colloid, preferably hydrophilic particle, said particle comprising a polyelectrolyte on its surface, wherein a cell-interacting entity is covalently bound to said polyelectrolyte. According to an embodiment, an active principle is associated or administered together with the colloid particle. Preferably, the particle is a chitosan-based nanoparticle having a negative zeta potential. Remarkably, particles of the present invention allow for efficient targeting of immune cells. In the field of vaccination, this can be exploited to produce a strong and durable immune response against antigens associated with the particles and/or particle solution.

The inventors of the present invention provided particles comprising a ligand bound to the particle, wherein said ligand preferably targets the particle to immune cells, in particular dendritic cells and/or macrophages. The ligand is preferably unlikely to induce an immune response against itself and binds to a receptor on the surface of the targeted cells. If a natural ligand of a receptor is used, in contrast, for example, to an antibody that may also have an affinity for the receptor and/or the targeted cell, the targeting is found to be effective.

Accordingly, in first aspect, the present invention provides a particle comprising, at its surface, a cell-interacting entity. Preferably, the cell-interacting entity is a ligand covalently or otherwise bound to the particle. Preferably, the particle harbours an active principle.

In a second aspect, the present invention provides a compound comprising a polyelectrolyte and, covalently or otherwise connected thereto, a cell-interacting entity.

In a second aspect, the present invention provides a particle comprising the compound of the invention. Preferably, the particle is a colloid particle.

In further aspects, the present invention provides a vaccine and/or a pharmaceutical composition comprising the compound and/or the particle of the invention.

In yet further aspects, the present invention provides the compound, the particles and/or compositions of the present invention for use in the treatment and/or prevention of a disease and/or an undesired condition, including the prevention and/or treatment of disease, for use as a medicament, for use as a vaccine, in particular a human or a veterinary vaccine.

In a further aspect, the present invention provides the compounds and/or particles of the invention in the treatment and/or prevention of immune disorders, in particular autoimmune disorders, allergic disorders, in particular allergy, inflammation, and in particular rheumatoid arthritis.

In another aspect, the present invention provides a method of treatment, for example a method of vaccination, the method comprising the step of administrating to an individual the compound of the invention, the particle of the present invention and/or the composition of the present invention.

In a further aspect, the present invention provides a process for preparing colloidal particles, the process comprising the steps of: providing a solution (a) comprising a first polyelectrolyte; providing a solution (b) comprising the compound of any one of the present invention, wherein said compound has a net charge that is inversed to the net charge of said first polyelectrolyte; providing a solution comprising both, said first polyelectrolyte and said compound, thereby obtaining a colloid particle.

In another aspect, the present invention provides a process for preparing colloid particles, the process comprising the steps of: (a) preparing colloid particles by mixing a polyelectrolyte and at least one selected from a polyanion and a polycation in an aqueous solution, wherein a polyanion is selected when said polyelectrolyte carries a net positive charge and a polycation when said polyelectrolyte carries a net negative charge, thereby obtaining colloid particles having a zeta potential; (b) adding the particles obtained under (a) to a solution comprising the compound of the present invention wherein said compound comprises, as a structural part of it, a polyelectrolyte that has a net charge that is inversed to the zeta potential of said colloid particles; thereby obtaining particles having a zeta potential that is inversed with respect to the zeta potential of the particles obtained under (a).

The present invention provides a further process for preparing colloid particles, the process comprising the steps of: (a) preparing colloid particles having a positive zeta potential by preparing a solution comprising a cationic polymer and comprising an anion; (b) adding the particles obtained under (a) to a solution comprising the compound of the present invention wherein said compound carries negative charges, thereby obtaining particles having a negative zeta potential.

Further aspects and preferred embodiments of the invention are provided in the appended claims.

In the drawings,

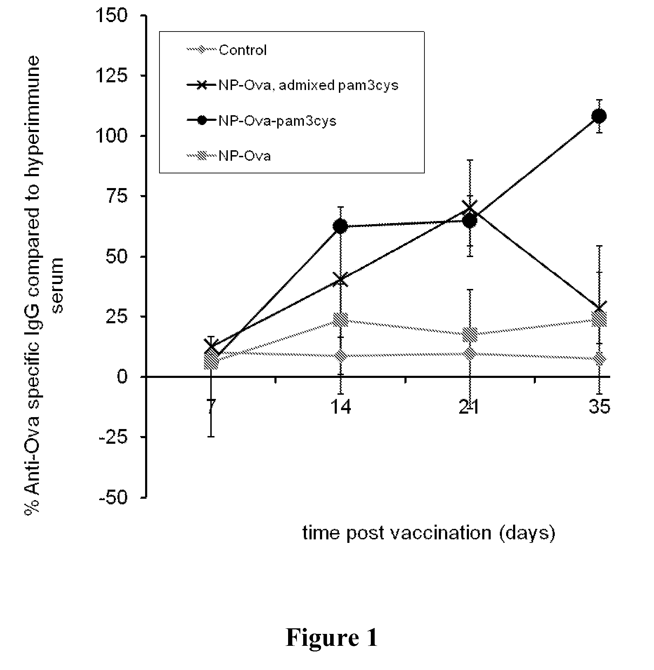

**Figure 1** shows levels of anti-ovalbumin antibodies produced in mice vaccinated with various compositions, including the vaccine composition of the present invention. Bright, filled diamonds are antibody levels of control mice, which did not receive any antigen. Filled circles are compositions according to the invention. Crosses are compositions, in which an adjuvant is admixed with vaccine particles comprising an antigen. Bright squares are compositions comprising only ovalbumin, the antigen, entrapped in colloid particles. Figure 1 shows that the particles of the present invention achieve a strong and long-lasting immune reaction, characterized by high levels of anti-ovalbumin antibodies over 35 days.

## Detailed Description of the Preferred Embodiments

The present invention concerns a compound comprising a cell-interacting entity covalently connected to a polyelectrolyte. The term "to comprise" or "comprising", for the purpose of the present specification, is intended to mean "includes amongst other". It is not intended to mean "consists only of".

According to an embodiment, the cell-interacting entity is selected from a targeting compound and/or an adjuvant.

According to the generally accepted definition, which is also valid for the purpose of the present specification, an adjuvant is a substance, which accelerates, prolongs, elicits and/or enhances an antigen-specific immune response when used in combination with a specific antigen. For example, recombinant proteins, which may be safe components of both human and veterinary vaccines are often weekly immunogenic and require adjuvants to make them effective vaccines. Generally, the adjuvant is characterized by the fact that it does not have a specific antigenic effect in itself.

Many adjuvants have an affinity for structures, for example cells, particular cell types, in particular immune cells, and/or receptors on the surface of cells. The affinity may be more or less specific, depending on the adjuvant. If there is a specific affinity for a particular receptor or family of receptors on a cell, the cell-interacting entity is a targeting compound.

More particularly, a targeting compound is a molecular entity that has an affinity with a target structure and therefore binds to and/or interacts with said target structure. The target structure generally is a cell, for example a protein on the surface of the cell, such as a receptor.

For the purpose of the present specification, the terms "bind to" and "having an affinity with" preferably refers to specific binding and specific affinity. Furthermore, it preferably refers to binding and affinity under physiological conditions.

According to an embodiment, the cell-interacting entity is a targeting compound and at the same time possesses adjuvant properties as defined above.

According to a further embodiment, the cell-interacting entity is an immune cell-targeting compound.

According to an embodiment, the cell-interacting entity is a ligand having a binding affinity to a cell-surface receptor.

According to yet a further embodiment, the cell-interacting entity is a ligand binding to a receptor of innate immune cells.

Below, compound classes and specific examples of structures functioning as cell-interacting entities and/or adjuvants for the purpose of the present invention are provided.

Generally, the cell-interacting entity may be an organic compound or an organometallic compound.

An overview of vaccine adjuvants can be found in the review by Frederick R. Vogel and Michael F. Powell, "A Compendium of Vaccine Adjuvants and Excipients", chapter 7, pp 141-227 in "Vaccine Design, the Subunit and Adjuvant Approach", Pharmaceutical Biotechnology, Vol 6, Eds. Michael F. Powell and Mark J. Newman, Plenum Press, New York and London, 1995.

Cell-interacting entities such as adjuvants and/or cell targeting compounds include nucleotides, proteins, peptides, carbohydrates, in particular oligo- and polysaccharides, lipids such as fatty acids and their esters, surfactants, vitamin derivatives, phospholipids, and compounds combining different categories, such as lipopeptides, lipoproteins, liposaccharides, peptidosaccharides and the like.

Examples for nucleotides functioning as cell-interacting entities for the purpose of the present invention are the oligodeoxynucleotide CpG motif CpG ODN (5'-TCC ATG ACG TTC CTG ACG TT-3') and the poly-I:poly-C structures.

Examples for proteins and peptides are cytokines with adjuvant or immunotherapeutic potential, such as IL-1beta, IL-1beta 163-171 peptide (sclavo peptide), IL-2, IL-6, IL-12, INF-alpha. These cytokines can be used to target cells carrying receptors that are specific for one or more of them.

Examples for proteins and peptides, which are not cytokines, are cholera holotoxin, cholera toxin B subunit, LT-OA (*E. coli* labile enterotoxin protoxin), transferrin, lactoferrin, peptides carrying RGD sequences, peptides carrying tetra-, penta-, hexa- hepta-or greater lysine sequences, and subunits or derivatives thereof.

Examples for peptidosaccharide structures are Theramide™ and peptidosaccharide-lipid structures such ImmTher™ (N-acetylglucosaminyl-N-acetylmuramyl-L-Ala-D-isoGlu-L-Ala-glycerol dipalmitate), MTP-PE (N-acetyl-L-Ala-D-isoGlu-L-Ala-2-(1,2-dipatmitoyl-sn-glycero-3-(hydroxyl-phosphoryloxy)) ethylamide mono sodium salt), Murametide (N-acetyl-Mur-L-Ala-D-Gln-OCH$_3$), Muralpalmitine™ (N-acetyl-Mur-L-Thr-D-isoGln-sn-glycerol dipalmitoyl) or D-Murapalmitine™ (Nac-Mur-D-Ala-D-isoGln-sn-glcerol dipalmitoyl) and Threonyl-MDP (Termutide™, N-acetyl muramyl-L-Thr-isoGlu).

Examples for surfactants are the different Montanide™ types, such as Montanide™ ISA 50V, Montanide™ ISA206 and Montanide™ IMS 1312 and the different span® types, such as Span 85 (sorbitane trioleate), span® 80 (sorbitane monooleate), span® 65 (sorbitan tristearate), span® 60 (sorbitane monostearate) and span® 20 (sorbitan monolaurate).

Examples for saccharidic structures are the linear and branched beta-glucans (e.g. algal glucan, pleuran or oligomeric subunits of these polysaccharids), gamma inulin or its subunits and GMDP (N-acetylglucosaminyl-(betal-4)-N-acetylmuramyl-L-alanyl-D-isoglutamine).

Examples for lipids are Avridine® (2-[3-(dioctadecylamino)propyl-(2-hydroxyethyl)amino]ethanol) and stearyl tyrosine.

Examples of lipopeptides and lipoproteins are those based on a Pam$_3$Cys and Pam$_2$Cys core, which are discussed in more detail below, as well as those based on low-density lipoprotein and acylated low density lipoprotein.

An example for a liposaccharide adjuvant is BAY R1005 belonging to the class of N-(2-aminoacylamido-2-desoxy-hexosyl)-amide, -carbamate and -urea (EP0206037).

Examples for vitamin derivatives are Calcitrol (1,25-dihydroxycholecalciferol) and Retinol ((2E,4E,6E,8E)-3,7-dimethyl- 9-(2,6,6-trimethylcyclohex-1-enyl) nona-2,4,6,8-tetraen-1-ol).

The term organic compounds encompasses, of course, many if not most of the cell-interacting entities of the various compound classes as detailed above. Examples for low molecular weight organic compounds are DHEA (dehydroepiandrosterone), Imiquimod (1-(2-methylpropyl)-1H-imidazol[4,5-c]quinolin-4-amine), Loxoribine (7-allyl-8-oxoguanosine), products derived from the bark of the *Quillaja saponaria* Molina tree (such as Stimulon™, QS-21, Quil A, quillic acid derivatives) and S-28463 (4-amino-alfa,alfa-dimethyl-2-ethoxymethyl-1H-imidazo[4,5-c]quinoline-1-ethanol).

Examples for phospholipids are DMPC (Dimyristoyl phosphatidylcholine), DMPG (Dimyristoyl phosphatidylglycerol) and MPL® (3-O-desacyl-4'-monophosporyl lipid A).

Examples of cell-interacting entities that are virus-derived immunomodulators are Baypamun® (parapoxvirus-derived preparation), and peptides generated to contain sequences mimicking the binding sites of virus binding ligands for cellular receptors.

While the cell-interacting entity according to the present invention may have adjuvant properties, this does not need to be. Examples of cell-interacting entities that are not typically adjuvants are ligands on the surface of pathogens, for example viruses or bacteria, conferring to those pathogens the capacity for interacting with cells. Examples of cell-interacting entities encompassed by the present invention are peptides carrying RGD motifs; peptides carrying KKTK motifs; specific glycan structures containing sialic acid for binding to sialic acid-binding immunoglobulin superfamily lectin (Siglec) receptors (such as Neu5Ac$\alpha$2-6GalNAc$\alpha$ for binding to Siglec-15 on dendritic cells); beta-galactoside containing glycoconjugates for binding galectins; N-acetylglucosamine (GlcNac) containing structures for binding to C-type lectin receptors (such as the Gal($\beta$1-4)(Fuc($\alpha$1-3))GlcNAc and Fuc($\alpha$1-3)Gal($\beta$1-4)(Fuc($\alpha$1-3))GlcNAc determinants relating to the glycosphingolipids of *Schistosoma mansoni*). In addition, transferrin (Tf) can be employed for targeting to the Tf receptor, low density lipoprotein (LDL) for targeting to the LDL receptor, and similarly for the various growth receptors on dendritic cells or on other cells to be targeted.

According to an embodiment, the cell-interacting entity is a ligand binding to a receptor of cells, preferably to a receptor of cells of the immune system. For example, the cell-interacting entity is a ligand binding to a receptor of an antigen-presenting cell, for example a dendritic cell, a macrophage, or a B-lymphocyte. For example, the cell-interacting entity is a ligand binding to a receptor of cells of the innate immune system.

For example, in case that a promotion of an immune defence is desired, the cell-interacting entity preferably has an affinity for at least one receptor on immune cells capable of being activated, wherein the binding to the receptor leads to the maturation and the desired activation of the cells. Examples of such receptors are the Toll-like receptors, such as the TLR2/6 and TLR2/1 dimers, TLR4, TLR5 and TLR6 on dendritic cells. Certain lipopeptides, in particular glyceryl-cysteinyl derivatives discussed below in more detail, bind to one or other of the aforementioned TLR2 heterodimer receptors.

It is also possible to target, with the cell-interacting entity, receptors inside cells. In this case, the targeting of the immune system relies on the cell-interacting entity binding to the cell surface receptor, such as scavenger receptors, with which the cell-interacting entity associates. The internal TLR, expressed for example by dendritic cells, in particular the plasmacytoid dendritic cells, are an example. Said cell-interacting entities include by way of example CpG-ODN motifs targeting TLR9, RNA-based motifs targeting TLR3 (dsRNA) and TLR7 (ssRNA). In these cases, however, a further ligand may be necessary to enhance targeting of the dendritic cells in the first place.

If, on the other hand, it is not desired to activate an immune cell, it is possible to target non-activating receptors of immune cells or immune cells that are already activated, such as inflammatory phagocytes. This may be the case, for example, if the purpose is to deliver a drug, regulatory molecule such as a cytokine and/or a genetic sequence for down-regulating and control of inflammatory responses. This is the case, for example, when inflammatory arthritis, IBD or other inflammatory, allergic or autoimmune disease is to be treated. In such cases, the cell-interacting entity may be, for example, a C-type lectin expressed by said inflammatory cells, or an antagonist for a receptor expressed on said inflammatory cells, for example a TNF antagonist or soluble TNF receptor.

Of course, the present invention also encompasses the targeting of cells other than immune cells, for example in cases of a therapeutic treatment not involving direct modulation of immune system cell activity.

Preferred cell-interacting entities for the purpose of the present invention are lipopeptides, in particular glyceryl-cysteinyl derivatives. In these compounds, up to three lipidic moieties are bound to a glyceryl-cysteinyl moiety. Furthermore, a peptide chain may be linked to the glyceryl-cysteinyl moiety. Preferred lipidic moieties are esters formed out of acetic hexanoic acid, octanic acid, decanoic acid, lauric acid, myristic acid, oleic acid and most preferably of palmitic acid. The most preferred cell-interacting entities for the purpose of this invention is tripalmitoyl-S-glyceryl-cysteinylseryl-lysine$_4$ (commonly abbreviated as Pam$_3$Cys-Ser-(Lys)$_4$ or Pam$_3$Cys-SKKKK; herein simplified to pam3cys). The structural moiety Pam$_3$Cys-OH, a lipoamino acid, for application in vaccine technology is derived and/or isolated from the N-terminal part of lipoprotein relating to those found on Gram-negative bacteria. This structural moiety seems responsible for the activation of monocytes, macrophages and dendritic cells, and thus indirectly at least B, T-helper and T-cytotoxic lymphocytes. Pam$_3$Cys-OH derivatives have found application in the form of Pam$_3$Cys-Ser-(Lys)$_4$, as an immunostimulatory adjuvant in vaccines comparable to Freund's complete adjuvant. Presently known Pam$_3$Cys-OH derivative-based adjuvants, which all are preferred cell-interacting entities of the present invention, include: Pam$_3$Cys = tripalmitoyl-S-glyceryl-cysteinyl; Pam$_3$Cys-Ser (tripalmitoyl-S-glyceryl-cysteinylseryl); Pam$_3$Cys-Ser-Ser (tripalmitoyl-S-glyceryl-cysteinylserylseryl).

The structural moiety tripalmitoyl-S-glyceryl-cysteinyl contains two stereocenters. All stereochemical combinations and mixtures of stereochemical differing products can be used for the purpose of the present invention.

As the skilled person will appreciate, the above-cited exemplary molecules for cell-interacting molecules will need to be provided as a constituent, for example as a substituent for connecting to the polyelectrolyte. Accordingly, the exemplary molecules will in some cases have to be derivatised, at least to some extent, for connecting to the cell-interacting entity.

According to an embodiment, the cell-interacting entity is a hydrocarbon comprising 5-5000 carbons atoms (C5-C5000), wherein 1 or up to half of said carbon atoms may be replaced by heteroatoms. More preferably, the cell-interacting entity is a C10-C2000, even more preferably a C20-C1000, and most preferably a C30-C200 hydrocarbon, wherein one or more carbons may be replaced by heteroatoms. Preferred heteroatoms include O, S, P, N and halogens.

The various different classes of compounds given above are not intended to be a conclusive list, but are only examples for structures functioning as cell-interacting entities for the purpose of the present invention. The fact that these examples pertain to various and different classes of compounds illustrates the general applicability of the concept of the present invention. The skilled person will understand that any specific cell-interacting entity will be selected dependent on the cell type and/or organ to be targeted, and/or on the effect being sought.

Since the compounds of the present invention may be used as constituents of colloid particles of the present invention, and since these particles may comprise chitosan, the cell-interacting entity covalently bound to the polyelectrolyte is preferably different from chitosan. In some embodiments of the particle of the present invention, chitosan is a polyelectrolyte that is part of the particle, and, therefore, the cell-interacting entity covalently bound to the polyelectrolyte needs to be different from chitosan to obtain a desired, additional effect. This distinction is necessary, because chitosan can display adjuvant properties. Moreover, chitosan, or at least particulate structures containing chitosan, may show properties of a targeting compound, since the chitosan structure can itself interact with dendritic cell surfaces. These cell-interacting properties are generally weak compared with the targeting ligands such as Pam3Cys, and so forth as disclosed above. This means that the particle of the present invention comprises at least two cell-interacting entities, in case that chitosan is used as particle component, or as the polyelectrolyte part of the compound of the invention. These two cell-interacting entities are, for example, chitosan (as a component of the particle matrix) and the cell-interacting entity covalently bound to the polyelectrolyte in the compound of the present invention.

The compound of the present invention comprises a cell-interacting-entity covalently connected to a polyelectrolyte. As mentioned above, the interacting entity can be a targeting compound and/or an adjuvant. The "and" indicates that it can be both. Alternatively, the present invention also encompasses the possibility that two different entities, one being a targeting compound the other being an adjuvant without targeting characteristics or targeting characteristics which are minor relative to the targeting compound, wherein both are applied to the surface of the same or different particles.

A polyelectrolyte, for the purpose of the present specification, is a polymer or an oligomer carrying a plurality of charged groups. Accordingly, the term "polyelectrolyte" encompasses cationic polymers and anionic polymers and may be selected from these two. The presence and quantity of charged groups, however, depends on conditions such as the pH and/or salt concentration of a solution in which the poylelectrolyte is provided. Since the polyelectrolyte may be used in the preparation of colloid particles according to the present invention, the presence of charged groups relates to the conditions under which formation of colloid particles can occur, which will be described further below.

A polymer, for the purpose of the present specification is a compound having 10 or more moieties characterized by a repetition pattern of its backbone or limited differing building units in the backbone, such as more than 10 mono saccharide and/or amino acid moieties, for example. An oligomer, for the purpose of the present invention, comprises 3-9 repeated moieties, the terms mono- and dimer thus being defined, for the purpose of the present invention, as is conventional.

Anionic polyelectrolytes encompass anionic polysaccharides and anionic polypeptides. Examples of anionic polymers are alginate, hyluronate, chondroitin sulfate, heparin, dextran sulfate, dermatan sulfate, heparan sulfate, gellan gum, pectin, kappa, lamda and iota carrageenan, xanthan and derivatives of the aforementioned; sulfated, carboxymethylated, carboxyethylated or sulfoethylated varieties of glucans, glucosamino glucans including chitosan or xylans, glucan or xylan derivatives, glucosaminoglucans or glucosaminoglucan derivatives; proteins like collagen and keratose. All of these example anionic polymers are available from various commercial suppliers or can be synthesized by those skilled in the art using known methodology. Heparin and derivatives are disclosed in WO 2007/042572, which is expressly incorporated herein by reference. In particular, the section discussing heparin, starting from page 7, line 15 to page 8, line 17, is incorporated herein.

In WO 2007/031812, anionic polysaccharides are disclosed comprising carboxymethyl moieties, carboxy moieties and/or sulfate moieties. The entire reference of WO 2007/031812, and in particular the anionic complexing partners therein, are incorporated herein by reference.

Cationic polyelectrolytes may be selected from polyethylene imine, polyethylene imine derivatives, poly(methylene-co-guanidine), poly-L-lysine and chitosan, including derivatives of the aforementioned.

Chitosans are preferred cationic polyelectrolytes for the purpose of the present invention, in particular in the colloid particles described further below. Chitosans may differ in average molar mass, distribution of molar mass, degree of deacetylation, acetylation pattern, type of anionic counterion and purity. Regarding molecular size, chitosans with molar mass from 1'000 to 1'000'000 g/mol can be used in the particles of the invention. The lower end of this range (below molar masses of approximately 10'000 g/mol) includes molecules that are trivially referred to as oligochitosans and are characterized, with respect to chitosan of higher molar mass, by improved solubility in aqueous solutions at pH values higher than 6. Preferred molar masses of the chitosans used in the particles of the invention are from 1'000 to 10'000 g/mol and from 10'000 to 100'000 g/mol. Typically, chitosans will be present in amounts exceeding 10% of the weight of the particles of the present invention. Chitosans are produced from crustacean shells or by biotechnological processes from funghi. Commercial sources of chitosans are, e.g., Primex Ltd. (Iceland), Marinard Ltd. (Canada) or FMC Biopolymers (U.S.) as producers of crustacean-based chitosans, and Kitozyme Ltd. (Belgium) as producer for biotechnologically derived chitosan. Chitosans used in the compound and/or the particles of the present invention can also be chemically modified, for example on their hydroxyl or on their amino functionality. Such derivatized chitosans can be used instead or in combination with unmodified chitosans or other polycations or as polyanions if the chitosan is modified with anionic functionalities. Examples of moieties linked to the chitosan molecule are fluorescence markers such as fluorescein, anionic groups such as carboxymethyl, neutral synthetic small molar mass chains such as polyethylene glycol (PEG) chains and saccharides such as mono- or oligo-saccharides such as mannose and galactose. Modifications on the chitosan's amino functions can be executed in order to obtain secondary, tertiary or quaternary amines. The latter being of special interest as a pH independent positive charge can be integrated in the chitosan molecule. Prominent derivatives are the trialkyl chitosans, such as trimethyl chitosan, as disclosed, for example, in WO2006064331, which is expressely and totally incorporated herein by reference.

According to a preferred embodiment of the compound of the present invention, the said polyelectrolyte is an anionic polyelectrolyte comprising one or more functional chemical groups selected from a carboxylic acid group, a sulphate group, a sulfonate group, a phosphonate group, and combinations of two or more of the aforementioned, and wherein said cell-interacting entity is covalently bound to said polyelectrolyte optionally by way of said functional chemical group, optionally via a linker moiety.

The present invention also encompasses the possibility that the cell-interacting entity is covalently bound by a non-charged group, such as a hydroxy group (for example, of a monosaccharide moiety within a polysaccharide) or a thiol group, for example, to the polyelectrolyte.

Said linker moiety may be any hydrocarbon having about 1-1000, preferably 2-300, more preferably 2-30 carbons, which hydrocarbon may be substituted or unsubstituted and wherein one or more carbons may be replaced by one or more heteroatoms. As the skilled person will appreciate, a linker moiety generally provides the necessary functional groups that enable, on the one hand, the covalent binding of the linker moiety to the polyelectrolyte, and, on the other hand, the covalent binding of the linker moiety to the cell-interacting entity. Accordingly, amongst the huge list of potential linkers, a linker moiety is preferably selected in a way that the synthesis of the compound of the invention is facilitated, can be conducted under mild conditions and/or by the aid of readily available reaction components.

Preferred bond-types for connecting the cell-interacting entity to the polyelectrolyte, or one or both of the aforementioned to a linker, may be selected independently, for example, from an amide bond, an ester bond, in particular a carboxylic acid-ester bond, an ether-bond or an imide bond.

For the purpose of the present specification, reference to the covalent connection of the cell-interacting entity to the poylelectrolyte also encompasses the possibility that a linker moiety is present, even if the linker moiety is not always specifically mentioned. For example, expressions such as "substituted by the cell-interacting entity", "cell-interacting entity substituent", "covalent bond to the cell-interacting entity" and the like also encompass substitution by or bond to a linker-adjuvant-construct. Of course, the linker moiety is not mandatory and in certain situations, depending on the specific reaction components (polyelectrolyte, cell-interacting entity), a linker moiety may actually not be necessary.

Generally, in the compound of the invention, some of the charged groups of the polyelectrolyte may be transformed into covalent bonds to the cell-interacting entity substituents, for example. Only part of the charged groups is thus substituted by the cell-interacting entity. In this way, the polyelectrolyte substantially conserves its polyelectrolyte properties, which are required for particle formation. According to an embodiment, the degree of substitution is 0.5-20%, preferably 1-15% and more preferably 2-10%, for example 3-8%, in particular about 5%, meaning that the indicated percentage of charged groups of the polyelectrolyte are transformed to covalent bonds to the cell-interacting entity. According to another embodiment a lower degree of substitution is applied, which is 0.5-20‰, preferably 1-15‰ and more preferably 2-10‰. According to yet another embodiment, a still lower degree of substitution is used, preferably 0.5-20, more preferably 1-15 and more preferably 2-10 out of 10'000 charged groups are substituted.

According to a preferred embodiment, the polyelectrolyte covalently connected to a cell-interacting entity in the compound of the invention is an anionic polymer. Preferably, it is selected from alginate, hyaluronate and chondroitin sulfate. Alginates and hyaluronates carry carboxylic acid groups, which, under suitable pH conditions, are anionic. These groups may partly be substituted to carry a cell-interacting entity at the degrees indicated above. Chondroitin sulphate comprises sulphate and carboxylic acid groups, any of which can be selected to attach the cell-interacting entity. Preferably the cell-interacting entity is attached to the carboxylic acid group.

Accordingly, preferred compounds of the present invention are alginate/cell-interacting entity, hyaluronate/cell-interacting entity and chondroitin sulfate/cell-interacting entity. The preferred cell-interacting entity is pam3cys, the preferred compounds of the invention are alginate-pam3cys, hyaluronate-pam3cys and chondroitin sulfate-pam3cys, with the pam3cys being bound by one of its amine groups by an amide bond to the free carboxylic acid group of alginate, hyaluronate and/or chondroitin sulfate at a substitution degrees as indicated above.

The present invention also relates to a colloid particle. The particles of the present invention are preferably hydrophilic particles.

Colloidal particles of the present invention are preferably prepared in solution in a process referred to in the prior art by different terms. For example, in US 2001/0051189 the expressions "ionic cross-linking" and "ionic gelation" are used. Borges et al. (2005), International Journal of Pharmaceutics 299 (2005) 155-166, use terms as "precipitation" and "coacervation". Some of the above terms may suggest that covalent bonds are created during particle formation, which is not the case. Therefore, the present inventors prefer the term "polyelectrolyte complexation", which expresses accurately the fact that particles are formed through electrostatic forces.

In general, for "polyelectrolyte compelxation" to occur, at least two oppositely charged components need to be provided, wherein at least one of these two needs to be a polyelectrolyte, that is a polymer. It is also possible that both components are polyelectrolytes.

In other words, the colloidal particles of the invention comprise at least one polyelectrolyte, which forms a three-dimensional network (matrix) occupying the volume of the particle, and, an oppositely charged electrolyte, which does not need to be polymeric, but which should carry sufficiently opposite charges so as to allow for the particle formation by electrostatic forces in aqueous solutions. Without wishing to be bound by theory it is believed that, in an aqueous solution, at the least one polyelectrolyte interacts with at least one molecule (a polyion), which may also be a poylelectrolyte, carrying at least two, but preferably a plurality (two or more) charges opposite to the charges of the polyelectrolyte, so that a cluster or matrix of polyelectrolyte and the molecule (polyion) is finally obtained, the cluster forming the particle of the present invention. By adjusting conditions, such as concentration of the cationic and anionic species, level of agitation of the aqueous solution, particle size can be adjusted.

Therefore, in an embodiment of the present invention, said colloid particle comprises said compound of the invention and at least one further molecule carrying a plurality of charges, said further molecule possibly being a further polyelectrolyte, wherein said compound and said at least one further molecule are associated with each other by electrostatic forces.

According to an embodiment, said compound of the invention is a second polyelectrolyte, and wherein said colloid particle further comprises a first polyelectrolyte, wherein said first and second polyelectrolytes carry inversed net charges.

In principle, the colloidal particles do not exhibit a core-shell structure as do many other known encapsulation delivery systems, for example those obtained by formation of oil in water emulsions, or for example liposomes. In core-shell encapsulation systems, the particle comprises a core of the substance to be delivered, which is retained by or in a capsule wall, which surrounds or incorporates the substance to be delivered thus forming a shell that is basically impervious to the encapsulated material.

In contrast, in the colloidal particles of the present invention, a matrix comprising at least one colloid is provided. If a substance is to be delivered, for example an antigen as described further below, this substance is preferably retained and/or entrapped in the matrix and/or forms part of the matrix.

From the above the following terms will become apparent. Accordingly, the "matrix" refers to the bulk of the particle volume, which comprises at least one polyelectrolyte and at least one electrolyte of opposite charge. The expression "matrix-forming" is attributed to components that help building up the matrix of the particle during particle formation. The "matrix-forming" components thus take part in the "polyelectrolyte complexation" that results in the particles of the present invention. Matrix-forming components are substantially evenly distributed in the matrix of the particle. As will be described further below, the particles may also have a surface-modulating component, which generally is a surface-modulating polyelectrolyte.

Accordingly, the particle of the present invention comprises at least one matrix-forming anion and at least one matrix-forming cation, which contribute to complexing during particle matrix formation, and which are distributed across the three-dimensional network of the entire particle volume.

The matrix-forming, complexing anion may be provided, for example, by tripolyphosphate (TPP) as disclosed in US 2001/0051189. The matrix-forming anion may also be selected from ATP (adenosine triphosphate), ADP (adenosine diphosphate), or derivatives thereof, and sulphate. In cases where the matrix does not contain a matrix-forming anionic polyelectrolyte, the matrix-forming cation needs to be a polyelectrolyte.

The matrix-forming anion can be any anion containing a plurality of negative charges at the pH value at which particle formation occurs. Specific examples of useful anions include the sulphate anion, oligophosphates such as tripolyphosphate (TPP), nucleoside triphosphate including adenosine triphosphate (ATP), nucleoside diphosphates including adenosine diphosphate (ADP), poly-acrylic acid, poly-methacrylic acid, chondroitin sulphate, alginate, hyaluronate, dextran sulphate, heparin, heparan sulphate, gellan gum, pectin, kappa, lambda and iota carrageenan, xanthan, exudate gums, carboxymethyl cellulose, carboxymethyl amylose, carboxymethyl dextran, carboxymethyl chitosan and derivatives thereof; sulphated, carboxymethylated, carboxyethylated or sulphoethylated varieties of glucans or xylans, glucan or xylan derivatives, glucosaminoglucans or glucosaminoglucan derivatives; proteins like collagen and keratose. All of these example anions are available from various commercial suppliers or can be synthesized by those skilled in the art using known methodology. Preferred anions are adenosine triphosphate, tripolyphosphate, alginate, hyaluronate, chondroitin sulphate, carboxymethyl cellulose and dextran sulphate. Most preferred are tripolyphosphate, chondroitin sulphate, adenosine triphosphate and alginate. As the above list indicates, the matrix-forming anion may, of course, also be an anionic polyelectrolyte. The same polyelectrolytes may be used as are used in the compound of the present invention.

The matrix-forming cation may be selected from the cationic polyelectrolytes mentioned above, in the context of the polyelectrolyte of the compound of the present invention. Preferably, the cationic, matrix-forming polyelectrolyte is a chitosan or a chitosan derivative as described above.

The present invention may further comprise a surface-modulating polyelectrolyte. The surface-modulating polyelectrolyte may be used for modifying the surface properties of the colloidal particle of the present invention. Preferably, the surface modulating polyelectrolyte is an anionic polymer. Anionic polymers useful for modifying the surface properties have, in general, the purpose of providing a negative zeta potential to the particles. Particles with negative zeta potential tend less to aggregate in extracellular fluids. Furthermore, they do not exhibit the generally biologically incompatible properties of particles carrying many positive charges at their surface.

According to a preferred embodiment, the compound of the present invention is a surface-modulating polyelectrolyte.

The anionic polymers for modifying surface properties of the particle are preferably located close to the surface of the particle, in proximity to the positive charges of the cationic polyelectrolyte of the particle so as to render the overall charges on the surface more negative than positive. As will be detailed further below, the surface modulating anionic polyelectrolyte is preferably applied in a separate step, by adding particles having positive surface charges (a positive zeta potential) to a solution of the anionic poylelectrolyte so as to create a negatively charged surface.

The process of modifying surface characteristics by the anionic polyelectrolyte is again "polyelectrolyte complexation", that is a polymeric substance attaches itself through electrostatic forces onto and/or partially into the surface of the particle and thus modifies the zeta potential, for example. Given the polymeric nature of the polymeric surface modulating substance, the latter will generally only partially diffuse inside the particle and thus be predominantly located at the surface.

According to an embodiment, the compound of the present invention is a surface-modulating polyelectrolyte (preferably: a polyanion) provided at the surface of the particle and providing said negative zeta potential, while at the same time presenting the cell-interacting compound covalently connected to the surface-modulating polyelectrolyte.

According to an embodiment, the colloid particle comprises an active principle. According to a preferred embodiment said active principle is selected from antigens, immunomodulatory substances, for example allergens, cytokines, and growth factors, a functional protein in general, including enzymes and metabolical modulators, nucleic acids, vitamins and drugs. Two or more different active principles may be comprised in the particle. Depending on the size, a single molecule or a plurality of active principles may be provided.

Drugs, for example, may be encapsulated in the matrix of the particle of the present invention, if they have suitable hydrophilic groups and suitable structures. If a drug is generally hydrophobic and/or for other reasons not suitable for encapsulation by polyelectrolyte complexation in the matrix of the particle of the present invention, it is possible to use the above-described matrix to entrap particulate materials of appropriate surface charge, significantly smaller in size, preferably having a mean diameter that is by at least of a factor 5 smaller in size than the herein described colloid particle. Such particulate materials can be liposomes or inorganic particles or other colloidal particles carrying hydrophobic drugs. Accordingly, the present invention provides, in an aspect, colloid particles as defined herein, which encapsulate particulate materials comprising hydrophobic drugs.

According to an embodiment, the colloid particle of the invention comprises an antigen. The antigen is designed to elicit a desired immune response, for example the generation of antibodies having an affinity and/or specificity for the antigen, and preferably binding to the antigen under physiological conditions, and/or the generation of a cellular response. An antigen as defined herein may also be processed to be associated MHC molecules to stimulate the relevant antigen-specific T lymphocytes. This stimulation of antigen-specific T lymphocytes refers to both cytotoxic and helper T lymphocytes (MHC Class I or Class II). The immune response is preferably such that it treats and/or prevents an undesired condition, in particular a disease. Accordingly, the antigen is preferably a vaccine antigen, which renders the particle of the invention useful as a vaccine.

Antigens are not limited to a specific class of substances, but are generally defined by their ability to induce an immune response. In some cases, an immune reaction directed to the antigen (and entities presenting the antigen) only occurs if an adjuvant is also present, for example co-administered in combination with the antigen. Albeit this recognized functional definition of antigens, antigens usually comprise structural elements of at least one selected from a peptide, polypeptide, protein and a carbohydrate, in particular a polysaccharide. While the more complex molecular structures can elicit a more avid immune response, the above elements can form antigens, alone or particularly in combination, such as with glycoproteins. Furthermore, lipids can also be important constituents of antigens, particularly when in combination with other elements such as proteins (lipoproteins). Parts of viruses, bacteria and other micro-organisms, such as parasitic protozoa may function as antigen. Such parts include the coats (capsids or envelope proteins or glycoproteins for viruses, whether they be non-enveloped or enveloped viruses), capsules, cell walls, flagella, fimbrae and toxins of micro-organisms. Examples of vaccine antigens are the haemagglutinin glycoprotein of influenza virus, the core antigen of hepatitis B virus, the detoxified toxin (toxoid) of tetanus and diphtheria bacteria, and NcMAG1, NcROP2 and PDI of *Neosporum caninum*. It is also possible to employ whole inactivated virus as the vaccine "antigen", examples of which are influenza virus, tick-borne encephalitis virus, and measles virus among others.

Since the present invention is concerned with vaccines in general, it also encompasses vaccines for the treatment and/or prevention of cancer. Accordingly, the term "antigen", for the purpose of the present invention also encompasses tumour antigens, which are substances generally produced by or on tumour cells, which substances elicit, optionally in combination with an adjuvant, an immune reaction directed against the tumour cells.

According to a preferred embodiment, at least part or some of the active principle, for example, an antigen, is associated with said colloid particle. Preferably, the active principle is comprised in said matrix of the colloid particle. However, all or part of the active principle may also be otherwise and/or elsewhere associated to the particles, for example at the particle surface and/or in a solution comprising the particles.

The active principle may be covalently bound to a component of the colloid particle. Preferably, it is associated to the particle by electrostatic forces or other molecular interacting forces.

The active principle in or associated with the particle of the present invention may be a nucleic acid, such as DNA or RNA, as disclosed in US20070898057P, for example. The RNA can be, for example, non-replicating RNA, relating to messenger RNA (mRNA), or it can be self-replicating RNA such as replicon RNA. Nucleic acids may be targeted with the particle of the present invention for the purpose of gene therapy, for example.

The particles of the invention may be used in the prevention and/or treatment of a disease, for example, in the form of a vaccine. Such a vaccine may be a human or a veterinary vaccine. Examples of such vaccines include influenza virus, measles virus, rubella vaccine, HIV, yellow fever virus, hepatitis B virus, hepatitis C virus, tetanus vaccine, diphtheria vaccine, tuberculosis vaccine, Chagas disease vaccine, malaria vaccine, and others for human application. In the veterinary field, examples are foot-and-mouth disease virus, pseudorabies virus, porcine circovirus type 2, distemper virus, canine parvovirus, leptospirosis, *Actinobacillus pleuropneumoniae*, and others.

The particles of the present invention are particularly useful as vaccines. Solutions containing the particles may thus be used as vaccine compositions. In these solutions, which may be the direct result of particle formation in aqueous solutions described above, the antigen may be totally or partly be entrapped in the particle, but it may also be present totally or in part in the solution of the composition, depending on the preparation process of the particles and/or the conditions of particle formation.

Without wishing to be bound by theory, the present inventors believe that the particle comprising a cell-interacting entity covalently bound to its surface may function as a strong adjuvant for an antigen. Preferably, with the antigen being in close vicinity or even entrapped inside the particle, an immune reaction against the antigen will be stronger, prolonged and more specific than in prior art settings. When the cell-interacting entity is a targeting compound, an advantage is brought to facilitating the construction of a particle-based vaccine.

In the context of vaccination, the present invention is also concerned with vaccines based on nucleic acids encoding the antigens suitable for the vaccination. These nucleic acids can be either DNA or RNA, although the latter are preferred when targeted to dendritic cells, considering their greater propensity for translation in dendritic cells. Delivery of nucleic acid and in particular DNA-based vaccines can also target non-immune cells with higher nuclear division rates, such as muscle cells and epithelial cells, thus allowing transcription of the nucleic acid leading the antigen production. Such antigen can be released from the targeted cells to be endocytosed by dendritic cells, or the whole cell transcribing the nucleic acid vaccine can be cross-presented by the dendritic cells.

The particles and the compound of the present invention are also useful as medicaments, for example for therapeutic and immunotherapeutic application, as those mentioned above. Since the present invention provides a tool for targeting any active principle of interest to any potential cell target, in addition to targeting vaccines and immunotherapeutic compounds to for example dendritic cells and macrophages, the active principle can be selected according to the desired medical effect ans thus the desired cell being targeted for the purpose of promoting said desired medical effect.

Accordingly, the particles and/or the compounds of the invention are useful in the prevention and/or treatment of a human disease, for example in the prevention and/or treatment of cancer, and/or of a proliferative disease and/or an inflammatory disease. For example, the particles and/or the compound are useful for the prevention and/or treatment of intraocular inflammation and/or inflammatory bowel disease.

The particles and or the compound may also be used in the prevention and/or treatment of rheumathoid arthritis.

According to an embodiment, the particles and/or the compound of the present invention are used in the prevention and/or treatment of an autoimmune disease.

The particles and/or the compound of the invention may be used for the prevention and/or treatment of a disease selected from addison's disease, alopecia reata, ankylosing spondylitis, antiphospholipid antibody syndrome, autoimmune hemolytic anemia, autoimmune hepatitis, bullous pemphigoid, coeliac disease, Crohn's disease, dermatomyositis, diabetes mellitus type 1, goodpasture's syndrome, graves' disease, Guillain-Barré syndrome, Hashimoto's disease, idiopathic thrombocytopenic purpura, inflammatory bowel disease, lupus erythematosus, multiple sclerosis, myasthenia gravis, pemphigus vulgaris, pernicious anaemia, polymyositis, primary biliary cirrhosis, psoriasis, rheumatoid arthritis, Sjögren's syndrome, temporal arteritis, ulcerative colitis, uveitis, vasculitis, Wegener's granulomatosis, and combinations of two or more of the aforementioned.

Furthermore, the particles and/or compound of the invention may be used in the prevention and/or treatment of inflammatory diseases such as can be seen with the excessive inflammation following particular virus infections

The particles of the present invention may be administered in any suitable way of administration, such as parenterally, intranasally, orally, or intravaginally, in particular any route as is conventional for the vaccine and/or medicament being delivered. For example, the particles may be locally injected into target tissue or otherwise applied to reach such target tissue, for example by intradermal, subcutaneous, intramuscular, transcutaneous, intraperitoneal, intravenous, intra-articular, intra-ocular, intra-tumour, or applied to interact with mucosal immune organs and tissues, for example by intranasal or oral or intravaginal route.

The particles may be provided in the form of a vaccine and/or pharmaceutical composition, that is a formulation suitable to be administered to an individual. Such formulations may comprise stabilisers, buffers, fillers, cryoprotectors, conservatives, and/or further additives, for example. The formulation may be liquid or solid, for example in the form of a dried powder, a freeze-dried powder or a tablet. The skilled person will select suitable formulations in dependence of the condition to be treated and the way of administration.

The present invention relates to a process for preparing colloidal particles, in particular the particles of the present invention. Preferably, the particles are obtained by the process of "polyelectrolyte complexation" mentioned above.

Colloidal particles of the invention can be obtained readily by drop-wise addition of an aqueous solution comprising one component of the particles to an aqueous solution containing another component of opposite charge and agitation. No particular attention needs to be paid to the size of the droplets or the flow rate of addition of the first solution to the second solution. Formation of the particles of the present invention occurs spontaneously by polyelectrolyte complexation of the system's anionic and cationic components. Particle formation is visualized in the so-called "Tyndall effect" that can be seen by the human eye. The solvent system for the component solutions can be water or aqueous salt solutions. Conditions of pH can be varied depending on the type of polyelectrolyte (e.g. the type of chitosan) used and can cover physiological pH ranges. Chitosans of molar masses above approx. 10,000 g/mol require slightly acidic pH values, preferably between pH 3.5-6.6, whereas chitosan of molar mass below 10,000 g/mol have a wider pH range in complex formation, pH 3.5-7.5.

Within certain limits, water-miscible solvents can be present in the solution, for example, alcohols such as methanol, ethanol, 2-propanol, or N-butanol, can be present at concentrations of up to about 20% (v/v).

Chitosan polyelectrolyte complex formation has been extensively described in Berger et al. Structure and interactions in chitosan hydrogels formed by complexation or aggregation for biomedical applications. J. Pharm. Biopharm. 57 (2004), 35-52 and Agnihotri et al. Recent advances on chitosan-based micro- and nanoparticles in drug delivery. Journal of Controlled Release 100 (2004), 5-28.

According to an embodiment, the colloidal particle of the invention is obtained by polyelectrolyte complexation in an aqueous solution conducted at a temperature in the range of 1°C to 100°C and at a pH in the range of 3.5 to 9, preferably 4-8. Preferably, the temperature is 5-50°C, most preferably 10 to 35°C. As indicated, the specific condition may be selected so that the electrolytes present carry the required charges and polyelectrolyte complexation occurs.

The particles of the present invention are beneficial over other particles due to the mild conditions under which particles can be formed.

According to an embodiment, the process of the invention comprises the step of providing a solution (a) comprising a first polyelectrolyte. The first polyelectrolyte may be anionic or cationic, but is preferably cationic. Preferably the first polyelectrolyte is chitosan or a derivative of chitosan.

Solution (a) may be combined with a solution comprising a molecule carrying a plurality of charges that are inversed to the charges of the first polyelectrolyte, thereby achieving particle formation. If the first polyelectrolyte is cationic, the molecule may be anionic, for example a maxtrix-forming polyanion as defined above. In this way, colloid particles are formed, the matrix of which is constituted by said first polyelectrolyte and said molecule. If chitosan is used as a first poylelecrolyte, a particle having a positive zeta potential may thus be obtained.

In a further step, solution (a), or the colloid particles obtained from solution (a) above, may be combined with a solution (b) comprising the compound of the present invention, wherein said compound has a net charge that is inversed to the net charge of said first polyelectrolyte. For example, if the first polyelectrolyte is chitosan, solution (b) preferably comprises an anionic polymer. By adding solution (a) or the colloid particles obtained from solution (a) above to solution (b) under conditions conducive for particle formation (adjustment of pH, stirring), the particles of the present invention may be obtained. If the compound of the invention in solution (b) comprises an anionic polymer, a particle having a negative zeta potential is obtained.

The active principle, for example an antigen, if present, is preferably added to the solution at the moment when the matrix is formed. For this reason, the active principle is preferably soluble under the conditions of particle formation. If the active principle as such is not soluble, it may be chemically modified, for example by attachment of a hydrophilic group or molecule, to become soluble in the respective solution, or it may be chemically bound to the matrix forming materials, directly or by way of a liker as defined above. As mentioned above, it is also possible to encapsulate the active principle in capsules such as liposomes, which can be solubilised in the solution.

Accordingly, the active principle may be dissolved in solution together with a matrix-forming electrolyte preferably carrying the same net charge. In this way, precipitation of the active principle alone is prevented. For example, if the active principle is an oligo- or polypeptide carrying a net negative charge at the conditions of particle formation, it may be dissolved in a solution comprising a matrix-forming anion as described above, and then added to a solution comprising a matrix-forming cation, so that preferably at least some of the active principle is incorporated in the matrix of the particles. The particles so obtained may then be added to a solution comprising a surface-modulating poylelectrolyte, for example the compound of the present invention.

The number and order of steps that are performed to produce particles of the invention can be varied. For example, a solution containing one or more anions may be combined as described above with a solution of a chitosan or of a mixture of different polycations comprising chitosan, for example. Amounts of components are chosen such that particles with negative zeta potential result from polyelectrolyte complex formation. Another method is to combine a solution comprising an anion with a solution comprising a chitosan, or a mixture of different polycations comprising chitosan, such that colloidal particles of positive zeta potential are obtained. If necessary, an excess of uncomplexed chitosan can be removed by processes such as dialysis, ultrafiltration and centrifugation. Thereafter, the dispersion of particles of positive zeta potential is combined with a solution comprising one or more surface modulating anionic polyelectrolytes, for example the compound of the present invention, forcing conversion of the particles with positive zeta potential to particles with negative zeta potential. It is noted that the two or more polyanions that are incorporated in the final particles (in the matrix and on the surface) may be the same or may be different.

A variation of the previous method is to produce a first dispersion of colloidal particles with positive zeta potential by combining a solution of chitosan and a solution of one or more polyanions. After removal of excess chitosan, should there be an excess, the first dispersion is combined with a solution comprising an active principle with a plurality of negative charges molecule as defined above, to produce a second dispersion, still of positive zeta potential. This second dispersion is then added to a solution of one or more surface-modulating anionic polyelectrolyte, preferably the compound of the present invention, to force conversion to particles with negative zeta potential.

Since particle formation occurs in aqueous solutions, including aqueous solutions comprising other solvents in addition to water as indicated above, the polycations and anions described herein are preferably soluble in such aqueous solutions. The requirement of solubility is met, if at the temperature and further parameters of particle formation sufficient amounts of polycation or anion, for example a polysaccharide, are dissolved in a specific solution so that particle formation through electrostatic forces can occur.

Since the particle formation occurs in aqueous solutions and controlling the pH values at the different stages of the particle formation may determine the particles' final physico-chemical characteristics, including aqueous solutions comprising buffers for eased pH control, buffers as additional components may be present during the particle formation or may be added after forming the particles. Typical buffers are acetate, citrate, phosphate, carbonate, MOPS or HEPES.

It is noted that additional components can be added during or after particle formation. Examples of such additional components are multivalent cations such as calcium, uncharged polymers such as polyethylene glycol, or uncharged saccharides. Additional components may also include one or more further biologically active substances, for example antigens, adjuvants, nucleic acids, immunomodulatory substances, metabolical modulators, vitamins, cytokines, growth factors, in particular active principles as mentioned above. Such biologically active substances may be any biologically active substance, including small-molecule drugs or pro-drugs and therapeutic or otherwise biologically active peptides or proteins, provided that they are soluble in aqueous solutions at concentrations exceeding the concentrations at which they are therapeutically active or exert their other biological activity. Specific examples of such biologically active substances are NSAIDs, preferably NSAIDs belonging to the classes of salicylates, aryl alkanoic acids, 2-aryl propionic acids, *N*-aryl anthranilic acids, pyrazolidine derivatives, oxicams, coxibs and sulphonanilides.

Depending on conditions under which the particles of the invention are produced, colloidal particles in the nanometer to micrometer ranges can be produced. The most relevant parameters determining the condictions are the concentration of electrolytes, agitation, pH, and temperature. Preferred colloidal particles of the invention are nanoparticles having an average diameter of between about 10 and 1000 nm, preferably 20 to 500 nm.

The size and size-distribution of the particles of the present invention may most accurately be determined by taking representative electron microscopic photographs and by measuring the particle diameters from the photographs.

Different sizes of particles may be produced, by adjusting the conditions and parameters of the particle formation. Particles may also be subjected to fractionation, for example filtration, so as to obtain more specific particle size fractions adapted for a particular use or application of the particles. For example, the particles may have an average diameter that is smaller than 100 nm, in the range of 100-300nm, 200-500nm, 300-800nm or above 500nm, as preferred for a specific application.

The particles may be dried, for example by freeze-drying or by other drying techniques such as spray drying, for example.

With respect to dry weight, the particle according to a preferred embodiment of the present invention may comprise with respect to the two matrix forming components 60-95 wt.%, preferably 80-95 wt.%, more preferably 85-92 wt.% of a first polyelectrolyte, such as cationic polyelectrolyte, for example chitosan; 5 to 40wt.%, preferably 5-20 wt.%, more preferably 8-15 wt.% of a multivalently charged molecule, for example a matrix-forming anion.

With respect to the dry weight, the particle according to a preferred embodiment of the present invention may comprise with respect to the ratio between the matrix forming components and an active principle, of 50-99 wt.%, preferably 50-75 wt.%, more preferably 60-75 wt.% of matrix forming components; 1 to 50 wt.%, preferably 25-50 wt.%, more preferably 25-40 wt.% of an active principle as defined herein.

With respect to the dry weight, the particle according to a preferred embodiment of the present invention may comprise with respect to the ratio between the matrix forming components plus an optional active principle and an anionic, surface modulating polyelectrolyte, in particular the compounds of the present invention, of 1-70 wt.%, preferably 1-60 wt.%, more preferably 10-60 wt.% of matrix forming components plus the optional active principle; 30 to 99 wt.%, preferably 40-90 wt.%, more preferably 40-60 wt.% of a further polyelectrolyte, preferably an anionic, surface modulating polyelectrolyte, in particular the compounds of the present invention.

As mentioned above, the particles of the present invention preferably have a negative zeta potential. A negative zeta potential is determined by electrophoretic mobility measurements and represents a net negative surface charge of the particle.

The present invention as described herein may be modified while still falling into the general scope of the inventive principle. For example, as already indicated above, charges of the constituents of the particles may be inversed without changing the principle of the present invention. For example, instead of a chitosan, a negatively charged polymer may be used in the matrix of the particles and particle formation ("polyelectrolyte complexation") may be obtained by addition of a multivalent cationic molecule, which does not necessarily need to be polymeric (thus replacing tripolyphosphate, for example).

The invention now being generally described, it will be understood by reference to the following examples, which are included merely for the purpose of illustration of certain aspects of the present invention and are not intended to limit the invention in any way.

### **Examples**

#### Materials

Chondroitin sulfate type A, TPP (penta sodium triphosphate), sulfo-NHS (N-hydroxysulfosuccinimide sodium salt) and EDC*HCl (N-(3-dimethylaminopropyl)-N'-ethylcarbodiimide hydrochloride) were purchased at Sigma-Aldrich (Sigma-Aldrich, Germany) and used without further purification. Alginate was purchased at Stärke und Nagler (Stärke und Nagler, Switzerland imported from ISP, Scotland) and purified by Medipol (Medipol Ltd., Switzerland). Chitosan was purchased at Primex (Primex, Iceland) and purified by Medipol (Medipol Ltd., Switzerland). Ovalbumin (albumin from chicken egg white, article no A5503) was purchased at Sigma-Aldrich (Sigma-Aldrich, Germany) and used without further purification. The pam3cys, (tripalmitoyl-S-glyceryl-cysteinylseryl-lysine$_4$), was purchased at EMC (EMC Microcollections, Tübingen, Germany, product code L2000).

### **Example 1: Covalent Connection of the Pam3Cy Adjuvant to Alginate**

Pam3cys is covalently bound to alginate by an amide linkage between one of the pam3cys amine functions and the alginate's carboxylic acid functions. The amide linkage is established via the so-called carbodiimide reaction. Three different degrees of modification, low intermediate and high, were obtained by varying concentration of reaction partners as follows:

1.1 Low degree of modification:

Alginate was dissolved in water, 2ml of solution of 1 wt.% were prepared. To this solution 1ml sulfo-NHS 3 wt. % and EDC*HCL 0.0012 wt.% were added. After 20-25min pam3cys (0.1mg in 2ml $H_2O$) was added. This corresponds to a ratio of one pam3cys per 1635 carboxylic acid functionalities of the alginate.

1.2 Intermediate degree of modification

Alginate was dissolved in water, 2ml of solution of 1.5 wt.% were prepared. To this solution 1ml sulfo-NHS 3 wt.% and EDC*HCL 0.015 wt.% were added. After 20-25min pam3cys (1.2mg in 2ml $H_2O$) was added. This corresponds to a ratio of one pam3cys per 205 carboxylic acid functionalities of the alginate.

1.3 High degree of modification

Alginate was dissolved in water, 2ml of solution of 0.5 wt.% were prepared. To this solution 1ml sulfo-NHS 3 wt.% and EDC*HCL 0.06 wt.% were added. After 20-25min pam3cys (5mg in 2ml $H_2O$) was added. This corresponds to a ratio of one pam3cys per 16 carboxylic acid functionalities of the alginate.

The reaction mixtures 1.1-1.3 were stirred at room temperature overnight. The reaction mixture was transferred into a dialyse tube (Spectra/Por® Biotech Cellulose Ester Dialysis Membrane MWCO: 100,000 Spectrum Laboratories, Inc, USA) and dialyzed against demineralized water. The retentate was recovered by freeze drying.

Gravimetrical analysis of the sample with high degree of substitution (1.3) gave a yield corresponding to an approx. ratio of one pam3cys per 40 alginate's carboxylic acid functionalities.

## Example 2: Preparation of Colloid Particles of the Invention

Colloidal particles containing ovalbumin (OVA) as an antigenic compound, chitosan and chondroitin sulphate, the particles having a surface decorated with pam3cys-alginate were prepared according to the following procedure:

88mg of ovalbumin were dissolved in 10ml of a 0.1% solution of chondroitin sulphate. At room temperature, this solution was added drop-wise under agitation to a solution of 90ml of a 0.1% chitosan solution in aqueous HCl at approx. pH3.5. Opalescence appeared after the first added drops and became increasingly intense. After 1h of gentle agitation, the dispersion was filtered through a 1.2 $\mu$m filter (mixed cellulose ester membrane, Sartorius, Germany) and tangential flow dialyzed against a solution of aqueous solution of HCl of pH4 using a 0.2 $\mu$m tangential flow module (Vivaflow, polyethersulfone membrane, Sartorius, Germany). After dialysis, the dispersion was diluted by a factor of 4 with an aqueous solution of HCl of pH4. 20mL of the dispersion containing particles were added drop-wise to a solution of 20 mL of 0.05% pam3cys-alginate sodium salt conjugate (theoretical degree of substitution 1:200, see Example 1.2) in water at approx. pH8. The pH was monitored and adjusted by adding 0.1N NaOH to maintain the pH close to pH7.2. The zeta potential was measured at less than -10mV.

## Example 3: Preparation of Particles of the Invention Using Tripolyphosphate

Colloidal particles containing ovalbumin, chitosan and tripolyphosphate (TPP), the particles having a surface decorated with pam3cys-alginate were prepared according to the following procedure:

88mg of ovalbumin were dissolved in 10ml of a 0.1 % solution of TPP. At room temperature, this solution was added drop-wise under agitation to a solution of 90ml of a 0.1% chitosan solution in aqueous HCl at approx. pH3.5. Opalescence appeared after the first added drops and became increasingly intense. After 1h of gentle agitation, the dispersion was filtered through a 1.2 $\mu$m filter (mixed cellulose ester membrane, Sartorius, Germany) and tangential flow dialyzed against a solution of aqueous solution of HCl of pH4 using a 0.2 $\mu$m tangential flow module (Vivaflow, polyethersulfone membrane, Sartorius, Germany). After dialysis, the dispersion was diluted by a factor of 4 with an aqueous solution of HCl of pH4. 20mL of the dispersion containing particles were added drop-wise to a solution of 20 mL of 0.05% pam3cys-alginate sodium salt conjugate (degree of substitution about 1:200, see Example 1.2) in water at approx. pH8. The pH was monitored and adjusted by adding 0.1N NaOH to maintain the pH close to pH7.2. The zeta potential was measured at less than -10mV.

## Example 4: The Particles of the Invention in an *in vivo* Immunization Test

Vaccination protocol

Four or five NMRI mice (CRR Füllinsdorf) per group were vaccinated subcutaneously with 150ul doses of:

(i) PBS;

(ii) 10ug Ovalbumin formulated in 1:1 (v/v) PBS:Montanide ISA 206 (Seppic);

(iii) 10ug Ovalbumin encapsulated in chitosan nanoparticles, surface-decorated with alginate and admixed with pam3cys (50ug);

(iv) 10ug Ovalbumin encapsulated in chitosan nanoparticles, surface-decorated with pam3cys-alginate (particles of Example 3); or,

(v) 10ug Ovalbumin encapsulated in chitosan nanoparticles, surface-decorated with alginate.

Sampling

Mice were routinely bled weekly by lateral vein incision from the tail. Serum was collected by incubation of blood at 4°C overnight and then centrifugation at 3000 x g for 10 minutes.

ELISA Analytics

An indirect ELISA was performed to determine anti-Ovalbumin specific IgG antibody relative reactivity. Briefly, a Maxisorb 96-well plate (Nunc) was coated overnight at 4°C with 100ug/ml Ovalbumin (Sigma) in 50mM Bicarbonate: Carbonate buffer pH 9.6. Plates were washed 3 times with PBS containing 0.05% (v/v) Tween-20. Samples were diluted 1 in 1000 in PBS, 0.05% (v/v) Tween-20, 1% (v/v) dried-skimmed milk and incubated for 1hr at 37°C. In each test a hyperimmune standard serum was included for comparison. This was produced previously by repeated immunisation of a mouse with Ovalbumin. After 3 washes, 1 in 2000 peroxidase-conjugated Fab goat anti-mouse-IgG (Jackson) diluted in PBS, 0.05% (v/v) Tween-20, 1% (v/v) dried-skimmed milk was added and incubated for further 1hr at 37°C. After further washes, the substrate O-phenylenediamine (Sigma)-H2O2 was added for 15 minutes and absorbance at 450nm measured by a VersaMax spectrophotometer (Molecular Devices). Antibody titres expressed as % relative reactivity were calculated from OD450 values as follows:

$$\% \text{ relative reactivity} = \frac{\text{average (test serum)} - \text{average (background)} \times 100}{\text{average (hyperimmune control)} - \text{average (background)}}$$

The results can be seen in Figure 1. The particles of the present invention, shown as black circles, elicited a strong immune response, which both persisted over the 35 day observation period and even apparently increased over time. When nanoparticles were administered with the adjuvant (pam3cys) only admixed to the particle solution (group iii, black crosses) a less marked immune reaction was noted, which soon came down to levels of the group with which antigen (OVA) was administered in the complete absence of an adjuvant (group v, bright squares).

These examples show that the particles of the invention elicit a strong immune reaction which persists over a prolonged time if compared to antigenic particle compositions of the state of the art. Furthermore, the examples show that by the covalent connection of the adjuvant to the particle surface, the effect of the adjuvant is improved. These results show that the particles of the invention are suitable vaccine delivery systems.

**Claims**

1. A compound comprising a polyelectrolyte and, covalently connected thereto, a cell-interacting entity.

2. The compound of claim 1, wherein the cell-interacting entity is selected from a targeting compound and/or an adjuvant.

3. The compound of any one of claims 1 and 2, wherein the cell-interacting entity is an immune cell targeting compound.

4. The compound of any one of the preceding claims, wherein the cell-interacting entity is a ligand having a binding affinity to a cell-surface receptor.

5. The compound of any one of the preceding claims, wherein the cell-interacting entity is a ligand binding, under physiological conditions, to a receptor of innate immune cells.

6. The compound of any one of the preceding claims, wherein the cell-interacting entity is connected to the polyelectrolyte by way of a linker moiety, said linker moiety being a hydrocarbon having 1-1000 carbons, wherein one or more carbons may be replaced by one or more heteroatoms.

7. The compound of any one of the preceding claims, wherein the cell-interacting entity is a vaccine adjuvant designed to elicit and/or enhance an immune response of an individual to an antigen, when the cell-interacting entity is administered in combination with the antigen.

8. The compound of any one of the preceding claims, wherein the cell-interacting entity is a hydrocarbon comprising 5-5000 carbons atoms, comprising one or more heteroatoms.

9. The compound of any one of the preceding claims, wherein said polyelectrolyte comprises one or more functional chemical groups selected from a carboxylic acid group, a sulphate group, a sulphonate group, a phosphonate group, and combinations of two or more of the aforementioned, and/or wherein said cell-interacting entity is covalently bound to said polyelectrolyte by way of said functional chemical group, optionally via a linker moiety.

10. A colloid particle comprising the compound of any one of claims 1-9.

11. The colloid particle of claim 10, wherein the colloid particle comprises a matrix, and wherein an active principle is comprised in said matrix.

12. The colloid particle of any one of claims 10 and 11, further comprising an active principle, said active principle being selected from antigens, immunomodulatory substances including allergens, cytokines, and growth factors, functional proteins in general, including enzymes and metabolical modulators, nucleic acids, vitamins and drugs.

13. The colloid particle of any one of the claims 10-12, wherein said colloid particle has a negative zeta potential.

14. The colloid particle of any one of claims 10-13, wherein said colloid particle comprises at least one further molecule carrying a plurality of charges, said further molecule possibly being a further polyelectrolyte, wherein said compound and said at least one further molecule are associated with each other by electrostatic forces.

15. The colloid particle of any one of claim 14, wherein said further molecule is a second polyelectrolyte and wherein the polyelectrolyte of said compound is a first polyelectrolyte, wherein said first and second polyelectrolytes carry inversed net charges.

16. The particle of any one of the claims 10-15, wherein the polyelectrolyte of said compound is an anionic polymer, and wherein said colloid particle further comprises a cationic polymer, in particular chitosan.

17. The particle of claim 16, wherein said anionic polymer is a surface-modulating polyelectrolyte, which provides a negative zeta potential to said particle, and/or wherein said cationic polymer is a matrix-forming polyelectrolyte.

18. The particle of any one of claims 10-17, further comprises a matrix forming anion.

19. A pharmaceutical composition comprising the colloid particle according to any one of claims 10-18.

20. The particle of any one of claims 10-18 and/or the composition of claim 19 for use in the prevention and/or treatment of an undesired condition, including the prevention and/or treatment of a disease.

21. The particle of any one of claims 10-18 and/or the composition of claim 19 for use as a vaccine, in particular a human or veterinary vaccine

22. The particle of any one of claims 10-18 and/or the composition of claim 19 for use as a medicament.

23. A method of vaccination, the method comprising the step of administrating to an individual the colloid particle of any one of claims 10-18 and/or the composition of claim 19.

24. A process for preparing colloidal particles, the process comprising the steps of:

- providing a solution (a) comprising a first polyelectrolyte;
- providing a solution (b) comprising the compound of any one of claims 1-9, wherein said compound has a net charge that is inversed to the net charge of said first polyelectrolyte;
- providing a solution comprising both, said first polyelectrolyte and said compound, thereby obtaining a colloid particle.

25. A process for preparing colloid particles, the process comprising the steps of:

(a) preparing colloid particles by mixing a polyelectrolyte and at least one selected from a polyanion and a polycation in an aqueous solution, wherein a polyanion is selected when said polyelectrolyte carries a net positive charge and a polycation when said polyelectrolyte carries a net negative charge, thereby obtaining colloid particles having a zeta potential;
(b) adding the particles obtained under (a) to a solution comprising the compound of the present invention wherein said compound comprises, as a structural part of it, a polyelectrolyte that has a net charge that is inversed to the zeta potential of said colloid particles;

thereby obtaining particles having a zeta potential that is inversed with respect to the zeta potential of the particles obtained under (a).

26. A process for preparing colloid particles, the process comprising the steps of:

(a) preparing colloid particles having a positive zeta potential by preparing a solution comprising a cationic polymer and comprising an anion;
(b) adding the particles obtained under (a) to a solution comprising the compound of any one of claims 1-9, wherein said compound carries negative charges, thereby obtaining particles having a negative zeta potential.

27. The process of claim 25 and/or 26, wherein step (a) further comprises the step of adding an active principle to the aqueous solution.

28. The process of any one of claims 26-27, wherein said cationic polymer is a chitosan or a derivative of a chitosan.

29. The compound of any one of claims 1-9, wherein the polyelectrolyte of said compound is different from chitosan.

**Figure 1**

Europäisches
Patentamt

European
Patent Office

Office européen
des brevets

# EUROPEAN SEARCH REPORT

Application Number

EP 08 17 1263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/034552 A1 (PROKOP ALES [US]) 21 March 2002 (2002-03-21) * paragraph [0014] - paragraph [0015] * * example 1 * * claims 1,2,6,8 * | 1-15 | INV. A61K9/50 A61K9/51 A61K9/16 A61K39/39 |
| X | WO 2007/146319 A (SYMPHONY MEDICAL INC [US]; UNIV CALIFORNIA [US]; FMC BIOPOLYMER AS [NO]) 21 December 2007 (2007-12-21) * claims 1,4,6,8 * * figure 3B * | 1-15 | |
| X | WO 98/49202 A (BIOMM INC [US]) 5 November 1998 (1998-11-05) * page 21, line 34 - page 22, line 8 * * claims 1,5,29 * | 1-15 | |
| X | WO 98/31382 A (UNIV NEW YORK [US]; NARDIN ELIZABETH [US]; MORENO ALBERTO [CO]) 23 July 1998 (1998-07-23) * page 6, line 17 - line 20 * * page 22, line 27 - page 23, line 6 * | 1-15 | TECHNICAL FIELDS SEARCHED (IPC) A61K |
| X | WO 2008/124483 A (SPECIGEN INC [US]; CAMPION BRIAN K [US]; SIEGEL MARVIN I [US]) 16 October 2008 (2008-10-16) * paragraphs [0026], [0054], [0066], [0067], [0097], [0098], [0135] * | 1-15 | |
| X | US 2008/044900 A1 (MOONEY DAVID J [US] ET AL) 21 February 2008 (2008-02-21) * paragraphs [0008], [0050], [0109] * * claims 1-4 * | 1-15 | |

-/--

The present search report has been drawn up for all claims

| Place of search | Date of completion of the search | Examiner |
|---|---|---|
| Munich | 17 July 2009 | Hedegaard, Anette |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

## EUROPEAN SEARCH REPORT

Application Number

EP 08 17 1263

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2001/051189 A1 (ALONSO FERNANDEZ MARIA J [ES] ET AL ALONSO FERNANDEZ MARIA JOSE [ES] E) 13 December 2001 (2001-12-13) * example 1 * * claims 1,6 * | 1-15 | |
| X | WO 95/02416 A (VIRUS RES INST [US]) 26 January 1995 (1995-01-26) * page 28, line 23 - line 28 * * claims 1,4,10 * | 1-15 | |
| X | US 2002/146825 A1 (UHLER MICHAEL D [US]) 10 October 2002 (2002-10-10) * claims 1-7 * | 1-15 | |
| A | VANDENBERG G W ET AL: "Factors affecting protein release from alginate-chitosan coacervate microcapsules during production and gastric/intestinal simulation" JOURNAL OF CONTROLLED RELEASE, ELSEVIER, AMSTERDAM, NL, vol. 77, no. 3, 13 December 2001 (2001-12-13), pages 297-307, XP004328582 ISSN: 0168-3659 * the whole document * | 1-15 | TECHNICAL FIELDS SEARCHED (IPC) |

The present search report has been drawn up for all claims

| Place of search | Date of completion of the search | Examiner |
|---|---|---|
| Munich | 17 July 2009 | Hedegaard, Anette |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.                    EP 08 17 1263

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2009

| Patent document cited in search report | | Publication date | Patent family member(s) | | Publication date |
|---|---|---|---|---|---|
| US 2002034552 | A1 | 21-03-2002 | US | 2003035838 A1 | 20-02-2003 |
| WO 2007146319 | A | 21-12-2007 | EP | 2032149 A2 | 11-03-2009 |
| | | | US | 2008138416 A1 | 12-06-2008 |
| | | | US | 2008069801 A1 | 20-03-2008 |
| | | | WO | 2007147014 A2 | 21-12-2007 |
| WO 9849202 | A | 05-11-1998 | AT | 275585 T | 15-09-2004 |
| | | | BR | 9809326 A | 04-07-2000 |
| | | | DE | 69826119 D1 | 14-10-2004 |
| | | | DE | 69826119 T2 | 15-09-2005 |
| | | | EP | 0977780 A1 | 09-02-2000 |
| | | | US | 6281341 B1 | 28-08-2001 |
| WO 9831382 | A | 23-07-1998 | AP | 1070 A | 19-05-2002 |
| | | | AU | 5931698 A | 07-08-1998 |
| | | | BR | 9806971 A | 14-03-2000 |
| | | | CA | 2278295 A1 | 23-07-1998 |
| | | | CN | 1244126 A | 09-02-2000 |
| | | | EP | 1007081 A1 | 14-06-2000 |
| | | | IL | 130917 A | 31-08-2005 |
| | | | JP | 2001509813 T | 24-07-2001 |
| | | | OA | 11185 A | 14-05-2003 |
| | | | ZA | 9800477 A | 14-10-1998 |
| WO 2008124483 | A | 16-10-2008 | US | 2008311145 A1 | 18-12-2008 |
| US 2008044900 | A1 | 21-02-2008 | AU | 2006326405 A1 | 21-06-2007 |
| | | | CA | 2632630 A1 | 21-06-2007 |
| | | | EP | 1960009 A2 | 27-08-2008 |
| | | | JP | 2009519042 T | 14-05-2009 |
| | | | WO | 2007070660 A2 | 21-06-2007 |
| US 2001051189 | A1 | 13-12-2001 | NONE | | |
| WO 9502416 | A | 26-01-1995 | AU | 690567 B2 | 30-04-1998 |
| | | | AU | 7328694 A | 13-02-1995 |
| | | | BR | 9407397 A | 05-11-1996 |
| | | | CA | 2167081 A1 | 26-01-1995 |
| | | | CN | 1128953 A | 14-08-1996 |
| | | | EP | 0792161 A1 | 03-09-1997 |
| | | | JP | 9500132 T | 07-01-1997 |
| | | | NZ | 269411 A | 30-03-2001 |
| US 2002146825 | A1 | 10-10-2002 | AU | 3128102 A | 03-06-2002 |
| | | | US | 2002197720 A1 | 26-12-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0206037 A
- WO 2007042572 A
- WO 2007031812 A
- WO 2006064331 A
- US 20010051189 A
- US 20070898057P A

### Non-patent literature cited in the description

- **Saupe et al.** Immunostimulatory colloidal delivery systems for cancer vaccines. *informa healthcare,* 2006, 345-54
- **Alving.** Liposomes as carriers of antigens and adjuvants. *Journal of Immunological Methods,* 1991, vol. 140
- **Seferian et al.** Immune stimulating activity of two new chitosan containing adjuvant formulations. *Vaccine,* 2001, vol. 19, 661-8
- **Borges et al.** Evaluation of the immune response following a short oral vaccination schedule with hepatitis B antigen encapsulated into alginate-coated chitosan nanoparticles. *European Journal of Pharmaceutical Sciences,* 2007, vol. 32, 278-290
- A Compendium of Vaccine Adjuvants and Excipients. **Frederick R. Vogel ; Michael F. Powell.** ''Vaccine Design, the Subunit and Adjuvant Approach'', Pharmaceutical Biotechnology. Plenum Press, 1995, vol. 6, 141-227
- **Borges et al.** *International Journal of Pharmaceutics,* 2005, vol. 299, 155-166
- **Berger et al.** Structure and interactions in chitosan hydrogels formed by complexation or aggregation for biomedical applications. *J. Pharm. Biopharm.,* 2004, vol. 57, 35-52
- **Agnihotri et al.** Recent advances on chitosan-based micro- and nanoparticles in drug delivery. *Journal of Controlled Release,* 2004, vol. 100, 5-28